# DEMANDE DE BREVET EUROPEEN

(11) **EP 1 591 085 A1**
(43) Date de publication de la demande: **02.11.2005**
(21) Numéro de dépôt: 05290634.4
(22) Date de dépôt: 23.03.2005
(51) Int. Cl.: A61F 2/46

(54) **Kit de préparation d'un mélange de comblement osseux, cartouche spécialement adaptée à ce kit et trousse d'implantation comportant ledit kit**

(30) Priorité: 30.04.2004 FR 0404633
(71) Demandeur: Dentak, 750011 Paris (FR)
(72) Inventeur: Kurc, Michel, 75011 Paris (FR)
(74) Mandataire: Orsini, Fabienne

(57) **Abrégé**

L'invention concerne un kit (100) de préparation d'un mélange d'un matériau de comblement osseux naturel ou synthétique et de sang ou de sérum physiologique.

Selon l'invention, il comprend une platine (110), une coupelle (120) et un tube (130 ;130') destiné à contenir le mélange, ouvert à ses deux extrémités (130A,130B ;130'A,130'B), la platine comportant un moyen de réception (111A,111B) d'une extrémité du tube positionné verticalement et la coupelle comprenant en son centre un orifice (121) qui communique avec un manchon (122) de montage amovible sur l'autre extrémité du tube.

## Description

### DOMAINE TECHNIQUE AUQUEL SE RAPPORTE L'INVENTION

La présente invention concerne un kit de préparation d'un mélange d'un matériau de comblement osseux naturel ou synthétique et de sang ou de sérum physiologique accompagné ou non d'un broyat d'os.

L'invention trouve une application particulièrement avantageuse dans les domaines de la parodontologie, de l'implantologie et de l'orthopédie où il existe une demande d'un tel mélange notamment pour combler une alvéole après extraction ou pour pallier à un déficit de volume d'os lors d'une implantation.

### ARRIERE-PLAN TECHNOLOGIQUE

Généralement, le matériau de comblement osseux se présente sous une forme sèche de granules ou de poudre conditionnée dans un flacon.

Actuellement, il n'existe pas de dispositif simple et réutilisable, adapté à la préparation et à l'application d'un mélange osseux dans une cavité osseuse d'un patient.

Usuellement, le praticien mélange dans une coupelle stérile les granules ou la poudre avec le sang du patient ou le sérum physiologique et éventuellement un broyat osseux pour former une composition homogène et malléable qu'il applique à la spatule dans la cavité osseuse considérée.

### OBJET DE L'INVENTION

Le but de la présente invention est de proposer un dispositif en kit utilisable plusieurs fois, simple d'utilisation, peu encombrant qui permet au praticien de réaliser facilement le mélange souhaité à partir des différents composants et de conditionner ce mélange de manière qu'il soit prêt à être injecté dans la cavité osseuse d'un patient.

Plus particulièrement, l'invention propose un kit de préparation d'un mélange d'un matériau de comblement osseux naturel ou synthétique et de sang ou de sérum, qui comprend une platine, une coupelle et un tube destiné à contenir le mélange, ouvert à ses deux extrémités, la platine comportant un moyen de réception d'une extrémité du tube positionné verticalement et la coupelle comprenant en son centre un orifice qui communique avec un manchon de montage amovible sur l'autre extrémité du tube.

D'autres caractéristiques non limitatives et avantageuses du kit selon l'invention sont les suivantes :
- ledit moyen de réception de la platine est pourvu de moyens de montage amovible adaptés à coopérer avec des moyens de montage complémentaires prévus sur le tube ;
- lesdits moyens de montage complémentaires prévus sur le tube sont adaptés à coopérer avec des moyens de montage amovible d'un conduit cylindrique d'une seringue d'injection ;
- ledit tube est un trépan muni de dents sur le pourtour de son ouverture avant et adapté à être monté de manière amovible, à l'aide desdits moyens de montage complémentaires, à l'extrémité d'un mandrin d'entraînement en rotation d'un dispositif de forage récupérateur d'os ;
- ledit moyen de réception de la platine comporte un joint d'étanchéité destiné à établir au niveau de l'ouverture du tube une jonction étanche entre le tube et ledit moyen de réception ;
- la platine comporte un moyen de réception du manchon de la coupelle, distinct dudit moyen de réception de l'extrémité du tube, adapté à obturer ledit orifice ;
- ledit moyen de réception du manchon de la coupelle est un plot globalement cylindrique dont le diamètre est légèrement inférieur au diamètre interne dudit manchon;
- ledit moyen de réception du tube est un plot globalement cylindrique dont le diamètre est légèrement inférieur au diamètre interne dudit tube ;
- lesdits moyens de montage sont des moyens à goupille ou des moyens à baïonnette ou des moyens d'encliquetage ou encore des moyens de vissage.

L'invention concerne également une cartouche de matériau de comblement osseux mélangeable, comportant une enveloppe tubulaire agencée de manière à former ledit tube du kit selon l'invention.

Enfin l'invention concerne une trousse d'implantation comprenant un coffret contenant au moins un trépan de prélèvement d'os, au moins une seringue d'injection dont le conduit d'injection comprend à son extrémité distale des moyens de montage amovible dudit trépan et un kit selon l'invention.

Avantageusement dans cette trousse d'implantation, la hauteur du coffret fermé par un couvercle est sensiblement égale à celle de la platine et de la coupelle engagée sur le moyen de réception de la platine dudit kit de préparation de sorte que ledit coffret fermé est apte à contenir ladite platine, positionnée à plat dans le fond du coffret, portant sur son moyen de réception ladite coupelle.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue d'ensemble en perspective éclatée d'un mode de réalisation du kit selon l'invention ;
- la figure 2 est une vue en perspective assemblée du kit de la figure 1 ;
- la figure 3 est une vue en perspective du kit de la figure 1 dans lequel la coupelle est montée sur le tube pour l'introduction du mélange dans ce dernier ;
- la figure 4 est une vue schématique en perspective d'une seringue d'injection d'os sur laquelle est monté le tube du kit de la figure 1 rempli d'un mélange ; et
- la figure 5 est une vue schématique en perspective de l'intérieur d'une trousse selon l'invention.

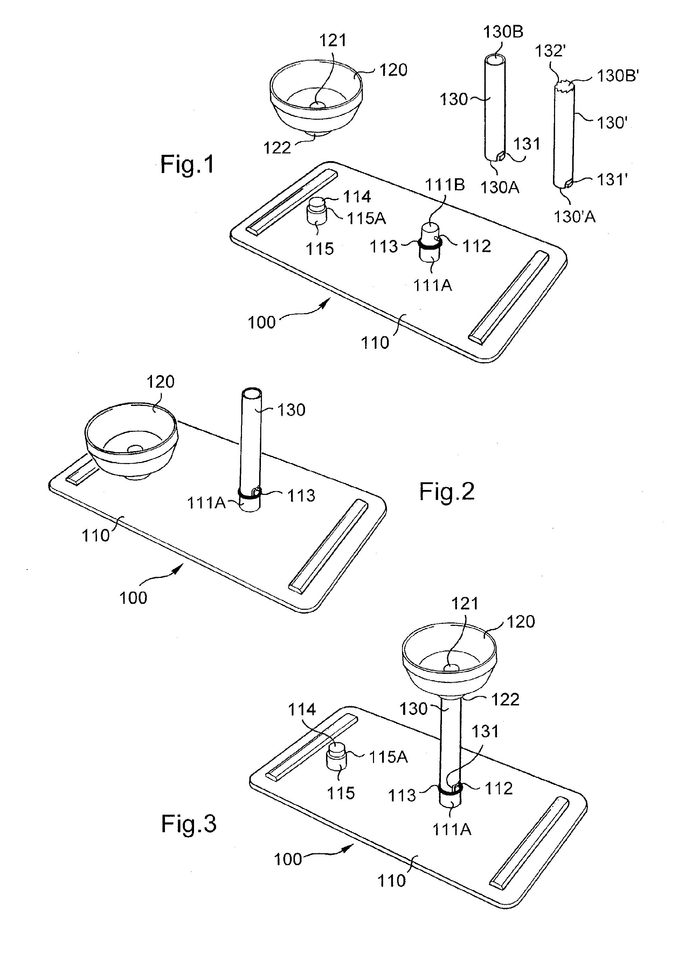

Sur les figures 1 à 3, on a représenté un dispositif en kit 100 de préparation d'un mélange d'un matériau de comblement osseux naturel ou synthétique et de sang ou de sérum et éventuellement d'un broyat osseux.

Ce kit 100 comprend une platine 110 se présentant sous la forme d'une plaque, une coupelle 120 et un tube 130 ;130' destiné à contenir le mélange, ouvert à ses deux extrémités 130A,130B ;130'A,130'B.

La platine 110 comporte un moyen de réception 111A,111B d'une extrémité 130A ;130'A du tube 130 ;130' positionné verticalement.

Ici, le moyen de réception est un plot globalement cylindrique qui s'élève perpendiculairement à la surface supérieure de ladite platine 110. Ce plot comprend une base 111A, dont le diamètre est légèrement inférieur au diamètre interne dudit tube 130,130', surmontée d'un téton 111B de plus petit diamètre. Un décrochement est formé à la jonction de la base 111A et du téton 111 B. Ce décrochement reçoit avantageusement un joint d'étanchéité 113 annulaire destiné à établir au niveau de l'ouverture 130A ;130'A du tube 130 ;130' une jonction étanche entre le tube 130 ;130' et ledit moyen de réception 111A,111B.

Préférentiellement, le moyen de réception 111A,111B est pourvu de moyens de montage amovible 112 adaptés à coopérer avec des moyens de montage complémentaires 131 ;131' prévus sur le tube 130 ;130'.

Ici, les moyens de montage amovible sont des moyens à baïonnette, comprenant sur le tube 130 ;130' deux gorges 131 ;131', positionnées symétriquement par rapport à l'axe dudit tube, et débouchant sur le bord de l'ouverture 130A ;130'A du tube, ces gorges 131 ;131' étant conformées pour permettre le montage à quart de tour de tiges 112 positionnées en correspondance sur le téton 111 B du plot porté par la platine 110.

Bien entendu, les moyens de montage amovible du tube 130;130' sur le plot peuvent, selon d'autres modes de réalisation non représentés, être des moyens à goupille comprenant un logement à l'extrémité arrière du tube destiné à coopérer avec une goupille prévu sur le plot, cette goupille étant actionnée élastiquement à l'aide d'un ressort.

Selon d'autres variantes de réalisation non représentées, on peut prévoir que ces moyens de montage sont du type à encliquetage ou à vissage.

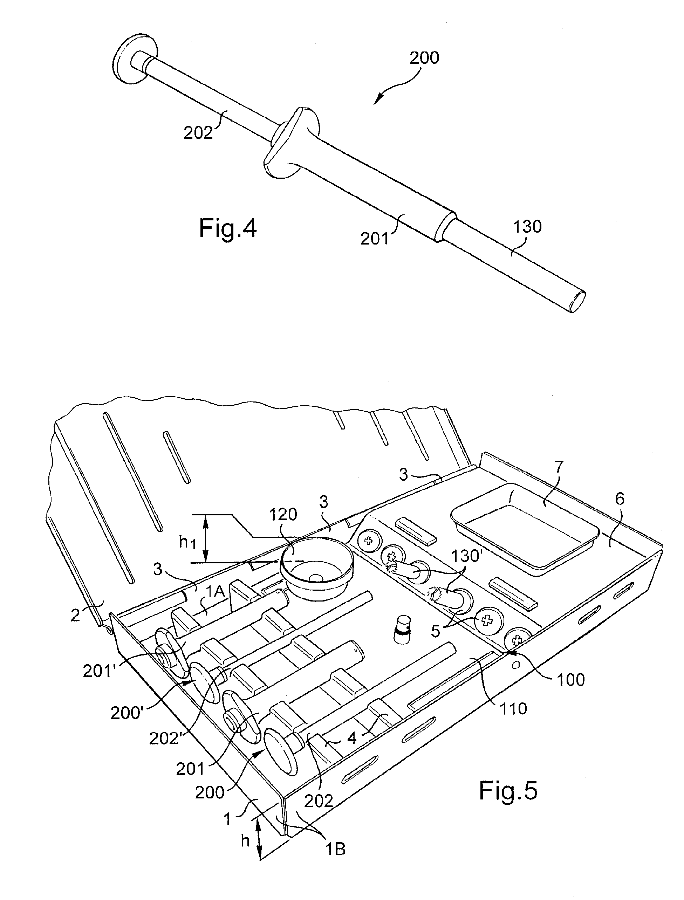

En outre, préférentiellement, comme le montre plus particulièrement la figure 4, lesdits moyens de montage complémentaires 131 prévus sur le tube 130 sont adaptés à coopérer avec des moyens de montage amovible d'un conduit cylindrique 201 d'une seringue d'injection 200. Le tube 130 prolonge axialement le conduit cylindrique 201 de la seringue d'injection d'os 200 qui comporte en outre une tige de piston 202 adaptée à coulisser dans le conduit cylindrique 201 prolongé par le tube 130 pour injecter dans le site receveur osseux d'un patient le mélange contenu dans ledit tube 130.

Comme le montrent les figures 1 et 5, selon un mode de réalisation du kit conforme à l'invention, ledit tube 130' est un trépan muni de dents 132' sur le pourtour de son ouverture avant 130'B et adapté à être monté de manière amovible, à l'aide desdits moyens de montage complémentaires 131', à l'extrémité d'un mandrin d'entraînement en rotation d'un dispositif de forage récupérateur d'os.

On citera, comme exemple de dispositif de forage, celui décrit dans le brevet FR 2 816 824. Ce dispositif de forage est remarquable en ce qu'il comprend un trépan équipé intérieurement d'une lame de broyage d'os. Cette lame de faible épaisseur s'étend longitudinalement selon l'axe du trépan et comporte une extrémité pourvue de deux bords tranchants formant une pointe qui s'étend dans le plan de l'ouverture avant du trépan. Cette lame fait partie d'un foret adapté à être monté de manière amovible sur le trépan par des moyens de montage à baïonnette, comme décrits précédemment, et comportant un mandrin d'entraînement en rotation, à l'extrémité duquel le trépan est monté de manière amovible, solidaire de ladite lame et s'étendant dans un sens opposé à cette dernière.

Selon une autre variante de réalisation particulièrement avantageuse de l'invention, le tube 130 du kit 100 constitue l'enveloppe tubulaire d'une cartouche de matériau de comblement osseux mélangeable.

Le tube 130 ;130' présente un diamètre externe compris entre environ 3 et 8 millimètres et une longueur de l'ordre de 20 millimètres pour une utilisation en chirurgie dentaire. Bien entendu, il peut présenter un diamètre et une longueur plus importants pour une utilisation en chirurgie osseuse orthopédique.

Par ailleurs, selon une caractéristique essentielle du kit 100 selon l'invention sa coupelle 120 comprend au centre de son fond un orifice 121 qui communique avec un manchon 122 de montage amovible sur l'extrémité ouverte 130B 130'B du tube 130 ;130' opposée à celle qui s'enfile sur le plot de la platine 110. À cet effet, le diamètre interne du manchon 122 est égal au jeu près au diamètre externe du tube 130 ;130'.

La coupelle 120 du kit 100 présente deux fonctions distinctes.

Elle forme un entonnoir permettant d'introduire proprement le mélange osseux à l'intérieur du tube 130 ;130 de sorte que celui-ci soit prêt à être injecté à l'intérieur du site receveur du patient.

À ce sujet, on remarquera que la coupelle 120 représentée sur les figures, présente une paroi interne légèrement conique et un fond plat mais selon une autre variante non représentée on peut prévoir que la conicité de la paroi interne de la coupelle soit plus accentuée et que son fond soit également en forme de tronc de cône débouchant dans l'orifice central 121.

La coupelle 120 forme également un récipient de mélange où le praticien peut déverser le matériau de comblement osseux naturel ou synthétique, le sang ou le sérum physiologique et éventuellement le broyat d'os, pouvant être prélevé à l'aide du tube 130' formant trépan, mélanger l'ensemble de ces composants avant de monter la coupelle 120 sur le tube 130 ;130' monté verticalement sur la platine 110 pour introduire ledit mélange dans le tube 130 ;130'.

Avantageusement, la platine 110 du kit 100 comporte un moyen de réception du manchon 122 de la coupelle 120, distinct dudit moyen de réception 111A, 111B de l'extrémité du tube 130 ;130', adapté à obturer ledit orifice 121. La coupelle 120 est alors positionnée sur ce moyen de réception pour être stockée ou pour être utilisée en tant que récipient de mélange avant d'être utilisée comme entonnoir sur le tube 130 ;130'.

Ici, ce moyen de réception est dans sa forme identique au moyen de réception 111A,111B du tube 130 ;130' sur la platine 110. Il est constitué d'un plot globalement cylindrique qui s'élève perpendiculairement à la surface supérieure de ladite platine 110. Ce plot comprend une base 115, dont le diamètre est légèrement inférieur au diamètre interne dudit manchon 121, surmontée d'un téton 114 de plus petit diamètre qui obture l'orifice 121. Lorsque le téton 114 obture l'orifice 121 de la coupelle 120 son extrémité libre arrive à effleurement du fond de la coupelle 120. Un décrochement 115A est formé à la jonction de la base 115 et du téton 114.

Ainsi, grâce à l'invention, le praticien peut prélever du broyat d'os chez le patient à l'aide du dispositif de forage décrit précédemment, puis déverser ce broyat d'os dans la coupelle 120 du kit 100 ou dans une autre coupelle plus grande, monter le tube 130' trépan sur la platine 110 prêt à recevoir le mélange, mélanger dans la coupelle 120 le broyat d'os avec un matériau de comblement osseux synthétique ou naturel et du sang ou du sérum physiologique, puis monter la coupelle 120 sur le tube 130' pour introduire le mélange ainsi obtenu dans ledit tube 130'. Le mélange est prêt à être injecté dans la cavité osseuse du patient. Cette injection s'effectue avantageusement à l'aide d'une seringue à injection à l'extrémité de laquelle le tube 130' rempli de mélange est monté.

En variante, le praticien peut déverser le contenu de la cartouche selon l'invention dans la coupelle 120 du kit 100 puis utiliser l'enveloppe tubulaire de cette cartouche en tant que tube 130 du kit 100 en la montant sur le moyen de réception correspondant de la platine 110, mélanger le matériau de comblement osseux à du sang ou du sérum physiologique puis monter la coupelle 120 sur le tube 130 pour introduire le mélange ainsi obtenu dans ledit tube 130. Le mélange est prêt à être injecté dans la cavité osseuse du patient. Cette injection s'effectue avantageusement à l'aide d'une seringue à injection à l'extrémité de laquelle le tube 130 rempli de mélange est monté.

Sur la figure 5, on a représenté une trousse d'implantation qui comprend un coffret 1, ici rectangulaire, fermé par un couvercle 2 monté à pivotement sur une paroi 1 B du coffret 1 par l'intermédiaire de charnières 3. Le coffret 1 et son couvercle 2 sont réalisés par exemple en matière métallique. Le coffret 1 contient au moins un trépan 130' de prélèvement d'os, ici deux trépans 130' de diamètre différent montés dans des sièges de réception 5, au moins une seringue d'injection, ici deux seringues d'injection d'os 200,200' chacune de ces seringues 200,200' comprenant une tige de piston 202,202' et un conduit d'injection 201,201' pourvu à son extrémité distale des moyens de montage amovible d'un desdits trépans 130' et un kit 100 selon l'invention.

Les tiges de piston 202,202' et les conduits d'injection 201,201' des seringues d'injection d'os 200,200' sont maintenus dans des encoches de deux réglettes 4 qui s'élèvent à partir du fond 1A du coffret 1.

En outre, il est prévu dans le coffret 1 une plateforme 6 comportant un trou de réception d'une coupelle de mélange 7, ici rectangulaire, de plus grande capacité que celle de la coupelle 120 du kit 100.

Ici les trépans 130' forment également tubes pour le kit 100 de préparation de mélange.

Avantageusement, dans cette trousse d'implantation, la hauteur h du coffret 1 fermé par son couvercle 2 est sensiblement égale à la hauteur h₁ de la coupelle 120 engagée sur le moyen de réception de la platine 110 dudit kit 100 de sorte que ledit coffret 1 fermé est apte à contenir ladite platine 110, positionnée à plat dans le fond 1A du coffret 1, portant sur son moyen de réception ladite coupelle 120.

L'ensemble des différents éléments du kit 100 sont réalisés en matériau biocompatible tel que par exemple de l'acier trempé inox. Le tube 130 dudit kit 100 peut lui être réalisé en un matériau synthétique stérilisable surtout lorsqu'il constitue l'enveloppe tubulaire de la cartouche selon l'invention.

La présente invention n'est nullement limitée aux modes de réalisation décrits et représentés mais l'homme du métier saura y apporter toute variante conforme à son esprit.

## Revendications

1. Kit (100) de préparation d'un mélange d'un matériau de comblement osseux naturel ou synthétique et de sang ou de sérum, qui comprend une platine (110), une coupelle (120) et un tube (130 ;130') destiné à contenir le mélange, ouvert à ses deux extrémités (130A,130B ;130'A,130'B), la platine (110) comportant un moyen de réception (111A,111B) d'une extrémité du tube positionné verticalement et la coupelle (120) comprenant en son centre un orifice (121) qui communique avec un manchon (122) de montage amovible sur l'autre extrémité du tube.

2. Kit (100) selon la revendication 1, **caractérisé en ce que** ledit moyen de réception (111 B) de la platine (110) est pourvu de moyens de montage amovible (112) adaptés à coopérer avec des moyens de montage complémentaires (131 ;131') prévus sur le tube (130 ;130').

3. Kit (100) selon la revendication 2, **caractérisé en ce que** lesdits moyens de montage complémentaires (131 ;131') prévus sur le tube (130 ;130') sont adaptés à coopérer avec des moyens de montage amovible d'un conduit cylindrique (201) d'une seringue d'injection (200).

4. Kit (100) selon l'une des revendications 2 ou 3, **caractérisé en ce que** ledit tube est un trépan (130') muni de dents (132') sur le pourtour de son ouverture avant (130B) et adapté à être monté de manière amovible, à l'aide desdits moyens de montage complémentaires (131'), à l'extrémité d'un mandrin d'entraînement en rotation d'un dispositif de forage récupérateur d'os.

5. Kit (100) selon l'une des revendications 1 à 4, **caractérisé en ce que** ledit moyen de réception (111A,111B) de la platine (110) comporte un joint d'étanchéité (113) destiné à établir au niveau de l'ouverture (130A ;130'A) du tube (130 ;130') une jonction étanche entre le tube et ledit moyen de réception.

6. Kit (100) selon l'une quelconque des revendications précédentes, **caractérisé en ce que** la platine (110) comporte un moyen de réception (114,115) du manchon (122) de la coupelle (120), distinct dudit moyen de réception (111A,111B) de l'extrémité du tube (130 ;130'), adapté à obturer ledit orifice (121).

7. Kit (100) selon la revendication 6, **caractérisé en ce que** ledit moyen de réception du manchon (122) de la coupelle (120) est un plot globalement cylindrique dont le diamètre est légèrement inférieur au diamètre interne dudit manchon.

8. Kit (100) selon l'une quelconque des revendications précédentes, **caractérisé en ce que** ledit moyen de réception (111A,111B) du tube est un plot globalement cylindrique dont le diamètre est légèrement inférieur au diamètre interne dudit tube.

9. Kit (100) selon l'une des revendications 2 à 4, **caractérisé en ce que** lesdits moyens de montage sont des moyens à goupille.

10. Kit (100) selon l'une des revendications 2 à 4, **caractérisé en ce que** lesdits moyens de montage sont des moyens à baïonnette.

11. Kit (100) selon l'une des revendications 2 à 4, **caractérisé en ce que** lesdits moyens de montage sont des moyens d'encliquetage.

12. Kit (100) selon l'une des revendications 2 à 4, **caractérisé en ce que** lesdits moyens de montage sont des moyens de vissage.

13. Cartouche de matériau de comblement osseux mélangeable, comportant une enveloppe tubulaire agencée de manière à former ledit tube du kit selon l'une des revendications 1 à 13.

14. Trousse d'implantation comprenant un coffret (1) contenant au moins un trépan (130') de prélèvement d'os, au moins une seringue d'injection (200 ;200') dont le conduit d'injection (201 ;201') comprend à son extrémité distale des moyens de montage amovible dudit trépan (130') et un kit (100) selon l'une quelconque des revendications 1 à 12.

15. Trousse d'implantation selon la revendication 14, dans laquelle la hauteur (h) du coffret (1) fermé par un couvercle (2) est sensiblement égale à celle de la platine (110) et de la coupelle (120) engagée sur le moyen de réception de la platine dudit kit (100) de sorte que ledit coffret (1) fermé est apte à contenir ladite platine (110), positionnée à plat dans le fond (1A) du coffret (1), portant sur son moyen de réception ladite coupelle (120).
